# EUROPEAN PATENT APPLICATION

(11) **EP 3 330 242 A1**
(43) Date of publication of application: **06.06.2018**
(21) Application number: 16830195.0
(22) Date of filing: 10.06.2016
(51) Int. Cl.: C07C 17/04, C07C 17/10, C07C 19/01, C07B 61/00

(54) **METHOD FOR PRODUCING HIGHER-ORDER CHLOROALKANE**

(30) Priority: 30.07.2015 JP 2015151246
(71) Applicant: Tokuyama Corporation, Shunan-shi, Yamaguchi 745-0053 (JP)
(72) Inventor: HOSAKA, Syunsuke, Shunan-shi Yamaguchi 745-0053 (JP); NISHIMOTO, Ryousuke, Shunan-shi Yamaguchi 745-0053 (JP)
(74) Representative: Beckmann, Claus
(86) International application number: PCT/JP2016/068021
(87) International publication number: WO 2017/018090

(57) **Abstract**

To provide a high-order chloroalkane production method in which a chloroalkane is subjected to a dehydrochlorination reaction in the presence of a Lewis acid catalyst composed of a metal chloride, the obtained chloroalkene is subjected to a chlorine reaction and the obtained reaction solution is purified by distillation, after a metal component derived from the catalyst is removed from the reaction solution, the reaction solution is distilled, thereby making it possible to reduce the amount of a deposit on the inner walls of equipment and pipes and to carry out distillation stably and continuously for a long time.

## Description

### TECHNICAL FIELD

The present invention relates to a chloroalkane production method accompanied by a dehydrochlorination reaction. More specifically, it relates to a method for obtaining a purified high-order chloroalkane by carrying out distillation after a chloroalkane is subjected to a dehydrochlorination reaction using a metal chloride as a catalyst and then to a chlorination reaction.

### BACKGROUND ART

Chloroalkanes are important as raw materials or intermediates for the manufacture of products such as agricultural chemicals, medicines, chlorofluorocarbon-replacing materials and fluorine compounds. For example, it is known that 1,1,1,2,3-pentachloropropane is dehydrochlorinated to obtain 1,1,2,3-tetrachloropropene from which trichloroallyl diisopropylthiocarbamate useful as a herbicide can be produced.

As the method of producing these chloroalkanes, there is known a three-stage reaction in which a chloropropane is obtained by adding carbon tetrachloride to an unsaturated compound having 2 carbon atoms (non-substituted or chlorine-substituted ethylene), a chloropropene is obtained by dehydrochlorinating the chloropropane and a high-order chloropropane having an increased number of chlorine substituents is obtained by chlorinating the chloropropene. Stated more specifically, JP-B 2-47969 discloses a method in which ethylene is reacted with carbon tetrachloride in the presence of a catalyst containing metal iron and trialkyl phosphate to obtain 1,1,1,3-tetrachloropropane, 1,1,1,3-tetrachloropropane is dehydrochlorinated to obtain 1,1,3-trichloro-1-propene or 3,3,3-trichloro-1-propene, and the trichloropropene is reacted with chlorine to obtain 1,1,1,2,3-pentachloro-1-propane. JP-A 2008-531474 discloses a method for obtaining 1,1,3,3-tetrachloro-1-propene by reacting carbon tetrachloride with vinyl chloride in the presence of a metal chloride containing metal iron and tributyl phosphate as a catalyst to obtain 1,1,1,3,3-pentachloropropane and subjecting 1,1,1,3,3-pentachloropropane to a dehydrochlorination reaction in the presence of a Lewis acid catalyst.

JP-A 2011-507877 discloses a method of producing 1,1,2,3-tetrachloro-1-propene by carrying out a dehydrochlorination reaction using iron chloride as a catalyst after 1,1,1,2,3-pentachloropropane is obtained by blowing a chlorine gas into the system while the dehydrochlorination of 1,1,1,3-tetrachloropropane is carried out by using iron chloride as a catalyst.

By the way, in general, the obtained high-order chloroalkane is an intermediate of the above-described raw material and supplied to the subsequent dehydrochlorination reaction step or fluorination step to be changed to a polychloroalkene, chlorofluoroalkane, chlorofluoroalkene, fluoroalkane or fluoroalkene. The obtained high-order chloroalkane is generally distilled to improve its purity before it is supplied to the subsequent step. It is known that when the high-order chloroalkane is supplied to the subsequent step while iron chloride which is the catalyst for the reaction remains, the dehydrochlorination reaction of a product of interest obtained by chlorination or fluorination proceeds, thereby causing the reduction of yield, and it is therefore desired to remove the iron chloride by the above distillation.

However, when distillation is carried out at a high temperature, a side reaction such as a polymerization reaction or dehydrochlorination reaction proceeds, thereby causing problems such as the reduction of yield and the corrosion of equipment.

Therefore, to prevent the above problems caused by the side reaction when purification is carried out by distillation, a measure such as low-temperature and low-pressure distillation is taken.

The dehydrochlorination reaction which is caused by the metal chloride catalyst residue can be prevented by deactivating the above catalyst. For example, JP-A 2004-534060 discloses that the above dehydrochlorination reaction in the distillation step is suppressed even in the presence of iron (III) such as iron chloride by adding a phenol compound to a polychloroalkane, thereby making it possible to carry out distillation without causing the reduction of yield by the side reaction.

Due to recent growing demand for chloroalkanes, it is becoming necessary to set a high distillation temperature or a distillation pressure close to normal pressure or to carry out distillation continuously for a long time in order to improve production efficiency. If a high distillation temperature is set, when a catalyst is deactivated by adding a phenol compound as described above, it is possible to suppress a dehydrochlorination reaction at the time of distillation caused by the residual catalyst or the dehydrochlorination reaction of a product of interest obtained by chlorination or fluorination in the subsequent step and further to mitigate a problem such as the corrosion of equipment caused by the produced hydrogen chloride.

However, it is difficult to completely prevent the dehydrochlorination reaction in a high-temperature distillation environment, the chloroalkene is gradually produced, and the polymerization reaction of the chloroalkene and the chloroalkane occurs. Therefore, the yield of the high-order chloroalkane of interest cannot be raised sufficiently, and a highly viscous material adheres to the inner walls of equipment and pipes due to the accumulation of the above polymers when distillation is carried out continuously for a long time, thereby causing a problem such as the blockage of these sites.

### DISCLOSURE OF THE INVENTION

It is therefore an object of the present invention to provide a high-order chloroalkane production method capable of improving the yield of a high-order chloroalkane and reducing the amount of a deposit on the inner walls of equipment and pipes at the time of distillation to carry out distillation continuously and stably for a long time when the high-order chloroalkane is produced by carrying out the dehydrochlorination reaction of a chloroalkane in the presence of a Lewis acid catalyst composed of a metal chloride (to be also referred to as "first reaction" hereinafter) and then the chlorination reaction of the obtained chloroalkene (to be also referred to as "second reaction" hereinafter) and purifying the obtained reaction solution by distillation.

The inventors of the present invention conducted intensive studies to solve the above problem and found that the above highly viscous material causing the blockage of the equipment is produced not only when anhydrous ferric chloride is used as the Lewis acid catalyst for the dehydrochlorination reaction of the chloroalkane but also when a metal chloride having the same activity is used.

They also found that the polymerization reaction of the chloroalkane and the chloroalkene proceeds by long-time operation at a high temperature to highly concentrate a compound having a higher boiling point than that of the high-order chloroalkane of interest (to be referred to as "high-boiling point material" hereinafter), such as a dimer or polymer, in the residual solution remaining in a distillation column, which is also one of the causes of increasing the production of the above highly viscous material, that is, the highly concentrated high-boiling point material forms an aggregate together with a metal component derived from the metal chloride added as the Lewis acid catalyst in the dehydrochlorination reaction of the above chloroalkane to produce the highly viscous material which adheres to the inner walls of equipment and pipes to cause a marked blockage problem.

Based on this finding, they found that the above problem can be greatly improved by removing the metal component derived from the catalyst from the reaction solution without deactivating the catalyst used in the above dehydrochlorination reaction (first reaction) after the chlorination reaction (second reaction) is carried out. That is, when the reaction solution containing no metal component obtained by removing the metal component derived from the catalyst is distilled, the proceeding of the above dehydrochlorination reaction can be suppressed as in the case where the catalyst is deactivated. They also found that even when a polymer is produced from the chloroalkene which is gradually produced despite of a severe environment, the aggregation of the chloroalkene does not proceed since the metal component is not contained in the above reaction solution and the chloroalkene does not grow into the highly viscous material with the result that the amount of a deposit on the distillation equipment can be reduced and distillation can be carried out stably without causing the blockage of the pipes. Thus, the present invention was accomplished based on this finding.

That is, the present invention is a high-order chloroalkane production method comprising the steps of:
carrying out the dehydrochlorination reaction of a chloroalkane represented by the following formula (1) in the presence of a Lewis acid catalyst of a metal chloride (first reaction) and chlorinating the obtained chloroalkene to produce a high-order chloroalkane represented by the following formula (2);
removing a metal component derived from the catalyst from the obtained reaction solution; and
distilling the reaction solution to isolate the high-order chloroalkane.

   Cₐ₊₁H_{b}Cl_{c+4} (1)

   wherein "a" is an integer of 2 to 4, "b" is an integer of 1 to 2a, and "c" is an integer of 0 to (2a-1), with the proviso that (b+c) is 2a.

   Cₐ₊₁H_{b-1}Cl_{c+5} (2)

   wherein "a", "b" and "c" are as defined in the above formula (1).

### MODE FOR CARRYING OUT THE INVENTION

The reactions of the above production method of the present invention will be described in detail hereinunder.

### (i) First reaction

In the present invention, the chloroalkane represented by the above formula (1) which is the raw material of the high-order chloroalkane is suitably selected according to the high-order chloroalkane of interest.

The high-order chloroalkane produced by the present invention, that is, the high-order chloroalkane represented by the above formula (2) is a chloroalkane having 3 to 5 carbon atoms and 4 to 11 chlorine atoms. A chloroalkane having 5 to 9 chlorine atoms is preferred. Specific examples of the chloroalkane include 1,1,1,2,3-pentachloropropane, 1,1,2,2,3-pentachloropropane, 1,1,1,2,2-pentachloropropane, 1,1,2,3,3-pentachloropropane, 1,1,1,2,2,3-hexachloropropane, 1,1,1,2,3,3-hexachloropropane, 1,1,1,2,2,3,3-heptachloropropane, octachloropropane, 1,1,1,2,3-pentachlorobutane, 1,1,1,2,3,3-hexachlorobutane, 1,1,1,2,4,4,4-heptachlorobutane, 1,1,1,2,2,4,4,4-octachlorobutane, 1,1,1,2,5-pentachloropentane, 1,2,3,3,5-pentachloropentane and 1,1,1,2,3,5,5-heptachloropentane. High-order chloroalkanes having a boiling point at 101.3 kPa of 150°C or higher out of the high-order chloroalkanes represented by the above formula (2) are preferred as the effect of the present invention can be attained efficiently, and 1,1,1,2,3-pentachloropropane, 1,1,2,2,3-pentachloropropane, 1,1,1,2,2-pentachloropropane and 1,1,1,2,2,3-hexachloropropane are particularly preferred as they are industrially useful compounds.

That is, the chloroalkane represented by the above formula (1) as the raw material in the present invention is a raw material compound for producing the high-order chloroalkane represented by the above formula (2), as exemplified by 1,1,1,3-tetrachloropropane, 1,1,2,3-tetrachloropropane, 1,1,2,2-tetrachloropropane, 1,1,1,2,3-pentachloropropane, 1,1,1,3,3-pentachloropropane, 1,1,1,2,2,3-hexachloropropane, 1,1,1,2,2,3,3-heptachloropropane, 1,1,1,3-tetrachlorobutane, 1,1,1,3,3-pentachlorobutane, 1,1,1,4,4,4-hexachlorobutane, 1,1,1,2,4,4,4-heptachlorobutane, 1,1,1,5-tetrachloropentane, 1,3,3,5-tetrachloropentane and 1,1,1,3,5,5-hexachloropentane. Particularly when the high-order chloroalkane represented by the above formula (2) is 1,1,1,2,3-pentachloropropane, 1,1,1,3-tetrachloropropane or 1,1,2,3-tetrachloropropane is selected and when the high-order chloroalkane represented by the above formula (2) is 1,1,1,2,2,3-hexachloropropane, 1,1,1,2,3-pentachloropropane or 1,1,2,2,3-pentachloropropane is selected.

The chloroalkane represented by the above formula (1) is dehydrochlorinated by the first reaction to produce a chloroalkene represented by the following formula (3).

Cₐ₊₁H_{b-1}Cl_{c+3} (3)

"a", "b" and "c" are as defined in the above formula (1). This chloroalkene is chlorinated by the second reaction to produce the high-order chloroalkane represented by the above formula (2).

The dehydrochlorination reaction of the chloroalkane is a reaction which proceeds in the presence of a catalyst in a batch or continuous system. As the catalyst, a Lewis acid catalyst composed of a metal chloride is used. Examples of the above metal chloride which functions as the Lewis acid catalyst is a chloride of a transition metal or a metal selected from the group XIII and group XIV metals, as exemplified by anhydrous aluminum chloride, anhydrous ferric chloride and lead chloride.

Out of these, anhydrous aluminum chloride and anhydrous ferric chloride are preferred as they are catalysts having high activity for the dehydrochlorination reaction, and anhydrous aluminum chloride is more preferred.

The amount of the catalyst in use is preferably 1.0 x 10⁻⁵ to 2.0 x 10⁻² mole, more preferably 5.0 x 10⁻⁵ to 1.0 x 10⁻³ mole based on 1 mole of the chloroalkane used in the reaction. In other words, it is preferred that the metal chloride should be used to ensure that its concentration falls within the above range when it is wholly dissolved. When the amount of the catalyst is too large, dimerization or polymerization proceeds due to a reaction between the chloroalkenes produced by the first reaction or a reaction between the chloroalkene and the raw material chloroalkane, whereby the selectivity of the high-order chloroalkane represented by the formula (2) of interest tends to lower. When the amount is too small, the reaction rate of the dehydrochlorination reaction tends to become low.

The metal chloride as the catalyst may be supplied into the reactor before or after the supply of the chloroalkane represented by the above formula (1), and a predetermined amount of the metal chloride may be supplied wholly at a time at the beginning, or in batches or continuously during the reaction, regardless of a batch reaction or a continuous reaction. Further, the metal chloride which is the catalyst may be dissolved in the chloroalkane outside the reactor and supplied into the reactor as a solution.

The reaction is accelerated more as the temperature becomes higher. The reaction temperature is suitably set according to the type of the catalyst in use, the type of the raw material chloroalkane and the type of the chloroalkene of interest. The reaction temperature is not particularly limited if it is not higher than the boiling point of the chloroalkene represented by the formula (3) but preferably 20 to 180°C, more preferably 30 to 150°C, much more preferably 50 to 120°C.

When the reaction temperature is too high for the type of the catalyst in use or the raw material chloroalkane, the reactivity of the obtained chloroalkene rises, whereby a by-product is produced by dimerization or a further reaction, and the selectivity of the chloroalkene of interest tends to lower. When the temperature is too low, the dissolution of the catalyst takes time and the reaction rate of the dehydrochlorination reaction tends to become low. When a reaction is carried out at a temperature close to the boiling point of the chloroalkene of interest, a gas phase part is preferably cooled and refluxed in consideration of the evaporation of the chloroalkene.

Since the reaction time differs according to the size of the reactor, the charge-in quantity of the raw material and the reaction temperature, it cannot be specified unconditionally but is preferably 0.5 to 12 hours, more preferably 1 to 6 hours. When the reaction is carried out continuously, the residence time is preferably 0.5 to 12 hours, more preferably 1 to 6 hours. When the reaction time is too short, the conversion rate of the chloroalkane becomes low and when it is too long, a side reaction proceeds and the selectivity of the chloroalkene becomes low.

For example, when anhydrous aluminum chloride is used as the catalyst and 1,1,1,2,3-pentachloropropane is used as the raw material chloroalkane to obtain 1,1,2,3-tetrachloro-1-propene and the reaction temperature is set to 50 to 120°C, the reaction time is about 1 to 6 hours. When anhydrous aluminum chloride is used as the catalyst and 1,1,1,3-tetrachloropropane is used as the raw material chloroalkane to obtain 1,1,3-trichloro-1-propene and the reaction temperature is set to 30 to 80°C, the reaction time is about 1 to 6 hours.

### (ii) Second reaction

The second reaction in the present invention is a chlorination reaction in which chlorine is added to the chloroalkene contained in the above first reaction solution. The high-order chloroalkane represented by the formula (2) is produced by this reaction. The reaction may be carried out in a batch or continuous system.

The metal chloride which has been used as the catalyst for the above-described dehydrochlorination reaction remains in the reaction solution obtained by the above first reaction. Although the metal chloride does not need to be deactivated as it functions as a catalyst in the chlorination reaction, it may be deactivated.

The case where the catalyst used in the first reaction is not deactivated will be described in detail. In this case, the second reaction can be carried out by supplying chlorine into the reaction solution without any special treatment of the reaction solution obtained by the above first reaction.

In this case, the reaction temperature which is suitably set according to the type of the catalyst used in the first reaction, the type of the chloroalkane used as the raw material and the type of the high-order chloroalkane represented by the formula (2) of interest is preferably -20 to 100°C, more preferably 0 to 80°C, much more preferably 10 to 60°C. When the reaction temperature is too high, the dehydrochlorination reaction of the formed high-order chloroalkane further proceeds, thereby reducing the yield of the high-order chloroalkane of interest. When the temperature is too low, the reaction rate becomes low.

Then, the case where the catalyst used in the first reaction is deactivated will be described in detail. When the catalyst is deactivated, the further dehydrochlorination reaction of the formed high-order chloroalkane is suppressed. Therefore, as compared with the above case where the catalyst is not deactivated, a higher reaction temperature can be set. When the catalyst is to be deactivated, the catalyst is preferably deactivated by adding an organic solvent having polarity to the reaction solution obtained by the first reaction. A phenol is used as the above organic solvent having polarity. More specifically, phenol and eugenol are preferred. The amount of the organic solvent having polarity is preferably not less than the amount (molar amount) of the catalyst used in the first reaction, more specifically, preferably 1 to 2 times, more preferably 1.1 to 1.5 times the amount (molar amount) of the catalyst. The addition of an excessive amount of the catalyst is uneconomical.

The reaction temperature when the catalyst has been deactivated is suitably set according to the type of the catalyst in use, the type of the raw material chloroalkane and the type of the high-order chloroalkane represented by the formula (2). It is preferably -20 to 150°C, more preferably 0 to 120°C, much more preferably 20 to 100°C. When the temperature is too high, a side reaction such as the further dehydrochlorination reaction of the formed high-order chloroalkane or dimerization proceeds, thereby reducing the yield of the high-order chloroalkane of interest. When the temperature is too low, the reaction rate becomes low.

In the second reaction, the amount of chlorine to be supplied is preferably not less than 0.9 mole, more preferably not less than 1 mole, much more preferably not less than 1.1 moles based on 1 mole of the chloroalkane represented by the above formula (1). When the amount is too large, the amount of wasted chlorine not contributing to the reaction becomes large. Therefore, it is preferably not more than 2.5 moles, more preferably not more than 2.0 moles based on 1 mole of the chloroalkane.

Chlorine may be supplied (into the reactor) at a time at the beginning of the second reaction. In this case, as the reduction of yield tends to be caused by intense heat generation and the further chlorination of the formed high-order chloroalkane due to the quick proceeding of the reaction, chlorine is preferably supplied gradually over a predetermined time. This supply time which differs according to the reaction temperature and the size of the reactor is generally 0.5 to 20 hours, preferably 1 to 10 hours, more preferably 1.5 to 5 hours. When chlorine is to be supplied over a long time, it may be supplied continuously or intermittently.

The reaction is preferably kept at the above temperature for a predetermined time after the whole amount of chlorine is introduced into the reactor. By doing so, the conversion rate can be improved. Stated more specifically, preferably, when the reaction is carried out in a batch system, the reaction time is 0.1 to 2 hours and when the reaction is carried out in a continuous system, the reaction solution is kept in another tank for 0.1 to 12 hours from the viewpoint of production efficiency.

The reaction time cannot be specified unconditionslly because it differs according to the size of the reactor, the charge-in quantity and the reaction temperature. In the case of a batch system, it is, for example, 0.5 to 22 hours, preferably 1 to 12 hours, more preferably 1.5 to 7 hours. When the reaction time is too short, the amount of generated heat becomes large, whereby cooling is required. In the case of a continuous system, after the reaction solution is caused to stay for 0.5 to 20 hours, preferably 1 to 10 hours, more preferably 1.5 to 5 hours, it should be transferred to another tank and kept for 0.1 to 12 hours while the temperature is kept at the above reaction temperature.

### (iii) Mode in which the first reaction and the second reaction are caused to proceed at the same time

In the present invention, the above first reaction and the above second reaction can be caused to proceed at the same time. Stated more specifically, chlorine is supplied into the reaction solution at the time of the above dehydrochlorination reaction to make the chlorination reaction of the chloroalkene proceed by adding chlorine to the formed chloroalkene, thereby obtaining the high-order chloroalkane represented by the above formula (2). The above mode in which the first reaction and the second reaction are caused to proceed at the same time is preferred in the present invention as a large amount of hydrogen chloride is caused to remain in the reaction solution after the second reaction. The reason that this mode is preferred will be described hereinafter. A description is subsequently given of the mode in which the first reaction and the second reaction are caused to proceed at the same time. These reactions may be carried out in a batch or continuous system.

The chloroalkane represented by the above formula (1) as the raw material and the metal chloride as the catalyst are as described in the first reaction (i). The amount of the catalyst in use is the same as that in the above first reaction. When the amount of the catalyst is too large, dimerization or polymerization caused by a reaction between the chloroalkene produced by the first reaction and the high-order chloroalkane produced by the second reaction proceeds in addition to the dimerization or polymerization of the chloroalkene described in the first reaction (i) or the raw material chloroalkane, whereby the selectivity of the high-order chloroalkane of interest tends to lower. When the amount of the catalyst is too small, the reaction rate of the dehydrochlorination reaction tends to become low as described above.

In this mode, the supply of the metal chloride as the catalyst into the reactor is the same as in the first reaction.

Then, the supply of chlorine into the reactor is preferably started after the metal chloride dissolves and the chloroalkene represented by the above formula (3) begins to be produced. In the reaction system, when chlorine is supplied before the metal chloride dissolves, not the first reaction but a substitution reaction caused by the chlorine of the chloroalkane represented by the formula (1) proceeds. For example, when the chloroalkane represented by the above formula (1) is 1,1,1,3-tetrachloropropane, a by-product such as 1,1,1,3,3-pentachloropropane is produced and the selectivity of 1,1,1,2,3-pentachloropropane of interest tends to become low. The dissolution of the metal chloride can be judged by the occurrence of a dehydrochlorination reaction. More specifically, it can be confirmed by the production of hydrogen chloride. Thus, in the mode in which the first reaction and the second reaction are caused to proceed at the same time, it is important that the amount of the metal chloride dissolved should be suitably adjusted.

The final supply amount, the supply system and the supply time of chlorine (Cl₂) are the same as those in the above second reaction.

Meanwhile, when the concentration of the chloroalkene in the reaction system becomes too high, a side reaction such as a reaction between the chloroalkenes or a reaction between the chloroalkene and the chloroalkane readily occurs as described above. Therefore, to suppress the side reaction involving the chloroalkene represented by the formula (3), it is preferred that the supply rate of chlorine (Cl₂) should be adjusted to keep the proportion of the chloroalkene represented by the formula (3) in the reaction system at preferably not more than 30 wt%, more preferably not more than 20 wit%, much more preferably not more than 10 wit% based on the total amount of the chloroalkane represented by the formula (1), the chloroalkene represented by the formula (3) and the high-order chloroalkane represented by the formula (2) in the reaction system.

It is preferred that the supply rate of chlorine (Cl₂) should be adjusted to keep the concentration of chlorine (Cl₂) in the reaction system at preferably not more than 10 wt%, more preferably not more than 5 wt%, much more preferably not more than 3 wt%, particularly preferably not more than 1 wt%.

In this embodiment, the first reaction is accelerated more as the reaction temperature becomes higher. When the supply of chlorine is small, the concentration of the chloroalkene represented by the formula (3) rises in the system and the dimerization of the chloroalkenes and a reaction between the chloroalkene and the chloroalkane proceed with the result that the amount of the by-product tends to grow. When the temperature is too low, the dissolution rate of the metal chloride and the reaction rate of the dehydrochlorination reaction tend to become low. Therefore, after the raw material chloroalkane is mixed with the metal chloride, the temperature of the reaction solution is kept at a range of preferably 0 to 100°C, more preferably 5 to 80°C, more preferably 10 to 60°C, particularly preferably 15 to 40°C before the start of supplying chlorine and during the supply of chlorine.

Also in this embodiment, like the above second reaction, after the whole amount of chlorine is introduced into the reactor, the reaction solution is preferably kept at the above temperature for a predetermined time. Although the reaction time in this embodiment cannot be specified unconditionally as it differs according to the size of the reactor, the charge-in quantity and the reaction temperature, in the case of a batch system, it is 0. 5 to 22 hours, preferably 1 to 12 hours, more preferably 1.5 to 7 hours. In the case of a continuous system, after the reaction solution is caused to stay for 0.5 to 20 hours, preferably 1 to 10 hours, more preferably 1.5 to 5 hours, it should be transferred to another tank and kept at the above reaction temperature for 0.1 to 12 hours.

### (iv) Removal of metal component

A description is subsequently given of the removal of a metal component derived from the catalyst and contained in the reaction solution obtained by the above reactions, which is the big feature of the present invention. In the present invention, the metal component derived from the catalyst contains a metal which has been added as the catalyst and is not particularly limited. Specific examples of the metal component include a metal chloride which has been added as the catalyst, and a compound and a metal complex which are produced from a reaction between the metal chloride and another component contained in the reaction solution.

Although the method of removing the above metal component is not particularly limited, an extraction method and an adsorption method may be employed. The extraction method which can remove the metal component effectively and is inexpensive is preferably used. The extraction method is a method in which two solvents immiscible with each other are used to remove the metal component by making use of a difference in metal component solubility between these solvents and called "liquid-liquid extraction method" or "solvent extraction method".

A description is subsequently given of the extraction method for removing the metal component. The types of the solvents used for extraction are not particularly limited if they can be separated from the high-order chloroalkane represented by the above formula (2) to form two phases and their solubilities for the metal component to be removed are higher than that of the high-order chloroalkane represented by the formula (2). Examples of the solvents include water and polyhydric alcohols. Out of these, water, ethylene glycol, propylene glycol, diethylene glycol and glycerin all of which have low affinity for high-order chloroalkanes are preferred.

The amount of each of the extraction solvents used in the above extraction method is preferably 0.01 to 1 part by mass, more preferably 0.03 to 0.3 part by mass, particularly preferably 0.04 to 0.1 part by mass based on 1 part by mass of the high-order chloroalkane represented by the above formula (2). When the amount of the extraction solvent exceeds the above range, its effect hits the ceiling and its cost becomes high. When the amount falls below the above range, it is difficult to separate the high-order chloroalkane and remove the metal component efficiently. After extraction, the extraction solvents in use may be recycled as extraction solvents after the metal component is removed by using purification means such as distillation.

When water is used as the above extraction solvent, the pH of water after extraction is preferably not more than 4, more preferably not more than 3, particularly preferably not more than 2. When the pH is at a neutral range from 4 to 7 or not less than 7, the metal chloride readily reacts with a hydroxide ion to become a metal hydroxide according to the type of the metal of the metal chloride in use. Metal hydroxides have very low solubility in a solvent as compared with metal chlorides in most cases and tend to precipitate as a solid. When this happens, it is difficult to carry out extraction with a solvent.

Therefore, to prevent the production and precipitation of the metal hydroxide, the pH of the extraction solvent is preferably at an acidic range. Even when a polyhydric alcohol is used as the extraction solvent, if it contains water like ethylene glycol, it tends to become a metal hydroxide as well. Therefore, it is preferred to prevent the production and accumulation of the metal hydroxide as well.

Therefore, when a solvent other than water is used as the extraction solvent, the concentration of an acid contained in the solvent after extraction is preferably not less than 10⁻⁵ mol/L, more preferably not less than 10⁻⁴ mol/L, particularly preferably not less than 10⁻³ mol/L. To this end, it is preferred to add an acid to the reaction solution or the extraction solvent in advance so that the pH of water when the extraction solvent is water or the concentration of the acid contained in the solvent after extraction when the extraction solvent is other than water falls within the above range. Stated more specifically, it is possible to adjust the concentration of the acid contained in the solvent after extraction by adding an acid such as hydrogen chloride or hydrogen fluoride to the reaction solution and/or adding a mineral acid such as hydrochloric acid, fluoric acid, sulfuric acid or nitric acid to the extraction solvent in advance.

By the way, the reaction solution to be supplied for extraction contains hydrogen chloride derived from the above dehydrochlorination reaction in the present invention. Since an acid such as hydrogen chloride or hydrogen fluoride contained in the reaction solution has higher solubility in the extraction solvent than the high-order chloroalkane, it parts from the high-order chloroalkane by the extraction of the present invention and moves into the extraction solvent.

In the present invention, the above acid concentration means a molar concentration when the acid contained in the solvent after extraction is converted into a monovalent acid. When a plurality of acids are contained, the above acid concentration is the total of the concentrations of these acids. The acid concentration is calculated by multiplying the concentration of an acid by the valence of the acid. More specifically, it is calculated from the total of the amount of the extraction solvent used in the extraction method and the amount of hydrogen chloride derived from the above dehydrochlorination reaction or the total of the acid concentration of hydrogen chloride derived from the above dehydrochlorination reaction and the acid concentration of the acid added to the reaction solution or the extraction solvent when the acid is added to the reaction solution or the extraction solvent. When the acid added is divalent sulfuric acid, the acid concentration becomes twice the concentration of sulfuric acid and when the acid added is trivalent phosphoric acid, the acid concentration becomes 3 times the concentration of phosphoric acid.

For example, when hydrogen chloride is added to the reaction solution to be supplied for extraction, the concentration of hydrogen chloride in the reaction solution is set to preferably 1.0 x 10⁻⁵ to 5.0 x 10⁻² part by mass, more preferably 1.0 x 10⁻⁴ to 1.0 x 10⁻² part by mass, particularly preferably 5.0 x 10⁻⁴ to 5.0 x 10⁻³ part by mass based on 1 part by mass of the high-order chloroalkane of the above formula (2).

Since the above hydrogen chloride, hydrogen fluoride and mineral acid have higher solubility in the extraction solvent than the high-order chloroalkane, they part from the high-order chloroalkane by the extraction of the present invention and move into the extraction solvent. As a result, the above acids rarely remain in the reaction solution from which the metal component derived from the catalyst has been removed and which is to be supplied into a distillation column. If the acids remain in the reaction solution, they can be easily removed by repeating the extraction method or the adsorption method.

By the way, the above-described first reaction is a reaction in which 1 mole of hydrogen chloride is produced when 1 mole of the chloroalkene is produced from the raw material chloroalkane. Therefore, the above-described mode (iii) in which the first reaction and the second reaction are caused to proceed at the same time is preferred in the present invention because a large amount of hydrogen chloride by-produced by the dehydrochlorination reaction is contained in the obtained reaction solution, thereby making it unnecessary to add an acid for solvent extraction or possible to reduce the amount of an acid to be added. Stated more specifically, it is preferred that all the by-produced hydrogen chloride should not be removed from the reaction solution obtained after the dehydrochlorination reaction and the fluorination reaction by nitrogen substitution operation and all or the above predetermined amount of hydrogen chloride should be caused to remain in the reaction solution, whereby the precipitation of the above metal oxide can be easily suppressed. In the case of the mode in which the first reaction and the second reaction are caused to proceed at the same time, the concentration of hydrogen chloride contained in the reaction solution which differs according to the reaction product and reaction conditions such as reaction time is preferably 1.0 x 10⁻⁵ to 5.0 x 10⁻² part by mass, 1.0 x 10⁻⁴ to 1.0 x 10⁻² part by mass, or 5.0 x 10⁻⁴ to 5.0 x 10⁻³ part by mass based on 1 part by mass of the high-order chloroalkane of the above formula (2). When the concentration of hydrogen chloride contained in the reaction solution does not fall within the above range, an acid gas such as hydrogen chloride or hydrogen fluoride, or mineral acid such as hydrochloride acid, fluoric acid, sulfuric acid or nitric acid may be added to the reaction solution, the extraction solvent or both of them.

The concentration of the metal contained in the reaction solution after the metal component has been removed is preferably not more than 5.0 x 10⁻⁶ part by mass, more preferably not more than 1.0 x 10⁻⁶ part by mass based on 1 part by mass of the high-order chloroalkane represented by the above formula (2).

Although the extraction system is not particularly limited, a one-stage extraction method, a multi-stage extraction method or a continuous counter-current method may be employed. A suitable method should be selected to ensure that the concentration of the metal contained in the reaction solution after the metal component has been removed falls within the above range.

### (v) Distillation

The reaction solution thus obtained after the metal component has been removed is distilled to separate and remove the chloroalkane represented by the formula (1) which is an unreacted raw material, the chloroalkene represented by the formula (3), a chloroalkene produced by the dehydrochlorination of the high-order chloroalkane represented by the formula (2) and other by-products, thereby making it possible to obtain the high-purity high-order chloroalkane represented by the formula (2). In the present invention, since the concentration of the metal component contained in the reaction solution to be distilled has been reduced to a certain value or less, the dehydrochlorination reaction hardly occurs during distillation and further, even when the high-boiling point material contained in the residual solution is highly concentrated at the bottom of a distillation column at a high temperature, the amount of a deposit on the distillation column can be reduced, whereby distillation can be carried out continuously and stably at a high temperature for a long time.

In the distillation step of the present invention, the high-purity high-order chloroalkene should be obtained, and distillation for the removal of a high-boiling point material having a higher boiling point than that of the high-order chloroalkane and distillation for the removal of a low-boiling point material having a lower boiling point than that of the high-order chloroalkane (to be referred to as "low-boiling point material" hereinafter) may be carried out. Although the order does not matter, distillation for the removal of the high-boiling point material is preferably carried out after distillation for the removal of the low-boiling point material from the viewpoints of thermal efficiency and the quality of the obtained high-order chloroalkane. When the above distillation for the removal of the low-boiling point material is carried out, the reaction solution from which the metal component has been removed is first distilled to remove the material having a lower boiling point than that of the high-order chloroalkane represented by the formula (2) as an effluent from the top of the column and collect the chloroalkane from which the low-boiling point material has been removed as a solution at the bottom of the column (residual solution). The metal component derived from the above-described catalyst is a high-boiling point material and continues to remain in the residual solution at a high temperature at the time of distillation for the removal of the high-boiling point material. Therefore, the effect of the present invention is especially obtained in distillation for the removal of the high-boiling point material. In general, the chloroalkane represented by the formula (1) which is the raw material and the chloroalkene represented by the formula (3) which is an intermediate have a lower boiling point than that of the high-order chloroalkane represented by the formula (2). When the boiling point of the extraction solvent is lower than that of the high-order chloroalkane represented by the formula (2), it is removed by distillation for the removal of the low-boiling point material.

In the present invention, in distillation for the removal of the high-boiling point material, the above collected solution at the bottom of the column is subjected to distillation for the removal of the high-boiling point material to collect the high-order chloroalkane represented by the formula (2) as a distillate from the top of the column and remove high-boiling point materials having a higher boiling point than that of the high-order chloroalkane, for example, the raw material chloroalkane, a dimer or polymer of the chloroalkene represented by the formula (3) and the metal component which could not be removed in the step of removing the metal component as the residual solution at the bottom of the column. When the boiling point of the extraction solvent is higher than that of the high-order chloroalkane represented by the formula (2), the extraction solvent is removed by distillation for the removal of the high-boiling point material.

In the present invention, any distillation column which is known in the industry may be used as the distillation column used for distillation, and a plate column or packed column is preferably used. For the above plate column, for example, a crossflow tray or shower tray may be used. When the above packed column is used, a known packing material such as Raschig ring or Lessing ring may be used. One distillation column or several distillation columns may be used.

The material of the distillation column is not particularly limited and may be a metal, glass or resin. There is a case where water and a small amount of hydrogen chloride are contained in the reaction solution from which the metal component has been removed. Therefore, it is preferred to choose a corrosion-resistant material such as glass, resin, Hastelloy or titanium as the material of a first distillation column. When several columns are used for distillation, hydrogen chloride and water are removed in the first column. The material of a distillation column after hydrogen chloride and water have been removed is not particularly limited.

The material of the packing material is not particularly limited and may be a metal, glass or resin. Since there is a case where hydrogen chloride and water are contained in the reaction solution to be supplied into the distillation column like the material of the above distillation column, it is preferred to choose a corrosion-resistant material such as glass, resin, Hastelloy or titanium. The material of the packing material used in a distillation column after hydrogen chloride and water have been removed is not particularly limited as well.

The purity of the high-order chloroalkane obtained by distillation is preferably not less than 98.0 %, more preferably not less than 99.0 %, much more preferably not less than 99.5 %. Although the distillation operation is not particularly limited, the temperature at the top of the column, the temperature at the bottom of the column, the inside pressure of the column, the reflux ratio and the number of stages should be adjusted to ensure that the purity of the high-order chloroalkane falls within the above range. In the present invention, the purity of the above high-order chloroalkane is a value obtained by separating an effluent from the distillation column by gas chromatography (GC) and determining with a hydrogen flame ionizing type detector (FID). The quantity of a component which cannot be detected with the above hydrogen flame ionizing type detector, for example, water can be determined with a Karl Fischer moisture titrate.

In the distillation operation for separating and removing the low-boiling point material, the temperature range at the bottom of the column is determined in consideration of the type of the high-order chloroalkane of interest, the type of the low-boiling point material, composition and pressure. For example, it is preferably 80 to 250°C, more preferably 100 to 200°C, particularly preferably 120 to 180°C.

In the subsequent distillation operation for separating and removing the high-boiling point material, the temperature range at the bottom of the column is determined in consideration of the type of the high-order chloroalkane of interest, the type of the high-boiling point material, composition and pressure as well. For example, it is preferably 100 to 300°C, more preferably 120 to 250°C, much more preferably 140 to 200°C, particularly preferably 150 to 180°C. The above temperature at the bottom of the column refers to the temperature of the residual solution at the bottom of the column, that is, the solution containing the concentrated high-boiling point material. When the temperature at the bottom of the column exceeds the above range, the high-order chloroalkane and other impurities tend to decompose, resulting in the reduction of yield. When the above temperature falls below the above range, it is necessary to reduce the pressure at the top of the column and the inside pressure of the column, thereby boosting equipment cost and operation cost, which is not efficient. In the distillation of the present invention, when the solution is taken out from the distillation column, heated outside and returned to the distillation column, the outside part for heating can be regarded as part of the distillation column, that is, the bottom of the distillation column.

In the present invention, when the temperature at the bottom of the column is set to the above range, the pressure at the time of distillation should be a value which ensures that the high-order chloroalkane represented by the formula (2) vaporizes and reaches the top of the distillation column and is determined by the type of the high-order chloroalkane, the temperature at the top of the distillation column and the type and size of the distillation column in use. When equipment cost and operation cost are taken into consideration, the pressure at the top of the column is set to preferably 1 kPa (abs) to 110 kPa (abs), more preferably 5 kPa (abs) to 50 kPa (abs), much more preferably 10 kPa (abs) to 30 kPa (abs).

The temperature at the top of the column should be a value which ensures that the high-order chloroalkane to be collected reaches the top of the column as a gas under the above pressure and that the high-order chloroalkane can be cooled properly not to be discharged to the outside of the distillation column. Therefore, it is determined in consideration of pressure at the time of distillation, the type of the high-order chloroalkane and the boiling point of the high-boiling point material contained in the residual solution. For example, when the high-order chloroalkane represented by the formula (2) is 1,1,1,2,3-pentachloropropane, 1,1,1,2,3-pentachloropropane can be collected at a high yield with almost no decomposition and side reaction by setting the temperature at the bottom of the column to 140 to 160°C, the temperature at the top of the column to about 120°C and the pressure at the top of the column during distillation to 10 kPa (abs).

Although additives may not be added at the time of distillation, a stabilizer may be added to suppress the thermal decomposition of the high-order chloroalkane of interest. Preferred examples of the stabilizer include phenols, phenols substituted by an alkoxy group and phenols substituted by an allyl group. Examples of the phenols substituted by an allyl group include o-allylphenol, m-allylphenol, p-allylphenol and 4-allyl-2-methoxyphenol (eugenol). These allyl-substituted phenols may be used alone or in combination.

The amount of the stabilizer to be added is preferably 1.0 x 10⁻⁶ to 2.0 x 10⁻⁴ part by mass, more preferably 1.0 x 10⁻⁶ to 2.0 x 10⁻⁵ part by mass based on 1 part by mass of the high-order chloroalkane represented by the formula (2) contained in the reaction solution to be distilled.

In the distillation step, when a phenol is added as the stabilizer at the time of distillation, it is preferably a phenol having a higher boiling point than that of the high-order chloroalkane represented by the formula (2). When it is so, the decomposition of the high-order chloroalkane can be prevented efficiently by carrying out distillation for separating and removing the high-boiling point material at the end of the distillation step.

The distillation system is not particularly limited. Batch system or continuous system may be used without restriction. According to the present invention, even when the temperature at the bottom of the column is kept high and the residual solution which is the high-boiling point material is highly concentrated, continuous operation can be carried out stably for a long time. Therefore, a remarkable effect is obtained by carrying out the continuous system.

In the distillation for the removal of the high-boiling point material of the present invention, the concentration of the high-boiling point material at the bottom of the column is preferably carried out to ensure that the content of the high-order chloroalkane represented by the formula (2) in the residual solution becomes 0.01 to 3 parts by mass, more preferably 0.05 to 2 parts by mass, much more preferably 0.1 to 1 part by mass based on 1 part by mass of the high-boiling point material.

When the extraction weight of the high-order chloroalkane represented by the formula (2) contained in the material to be removed exceeds the above range, the yield of the high-order chloroalkane of interest drops, thereby reducing production efficiency. When the extraction weight of the high-order chloroalkane represented by the formula (2) contained in the material to be removed falls below the above range, the viscosity of the residual solution increases, thereby making it difficult to remove it from the distillation column.

When a plate column is used, an extraction solution for each stage for removing part of the high-boiling point material may be added to the residual solution in distillation for the removal of the high-boiling point material.

The high-order chloroalkane obtained as described above is important as a raw material or intermediate for the manufacture of products such as agricultural chemicals, medicines, chlorofluorocarbon-replacing materials and fluorine compounds. For example, 1,1,1,2,3-pentachloropropane is used as the raw material of 1,1,2,3-tetrachloro-1-propene or 2-chloro-3,3,3-trifluro-1-propene, and 1,1,2,3-tetrachloro-1-propene is used as a raw material for the production of trichloroallyl diisopropylthiocarbamate which is useful as a herbicide or the raw material of 2-chloro-3,3,3-trifluoro-1-propene.

### EXAMPLES

The following examples are provided for the purpose of further illustrating the present invention but are in no way to be taken as limiting.

### Example 1

(1) 2,343 g of purified 1,1,1,3-tetrachloropropane having a purity of 99.5 % was fed to a 2,000-ml eggplant flask. Then, 0.61 g of anhydrous aluminum chloride was fed to the flask and stirred by setting the liquid temperature to 30°C. After it was confirmed that the solution turned blue and aluminum chloride dissolved, chlorine was caused to flow into the flask at a rate of 2,000 ml/min while the liquid temperature was kept at 25 to 30°C. After 1.5 hours, the flow rate of chlorine was changed to 1,000 ml/min. After 45 hours, the flow rate was further changed to 500 ml/min. After 30 minutes, an inflow of chlorine was stopped. The concentration of hydrogen chloride contained in the reaction solution at this point was 4, 000 ppmw based on the solution after the reaction. 170 g of water was added. A water layer (light layer) was separated by stopping agitation to obtain 2,865 g of a reaction solution (heavy layer). No solid was seen in the water layer and the pH of the water layer was not more than 1. When the reaction solution was analyzed by gas chromatography (to be abbreviated as GC hereinafter) and induction plasma emission analysis (to be abbreviated as ICP hereinafter), the conversion rate of 1,1,1,3-tetrachloropropane was 100 %, the selectivity of 1,1,1,2,3-pentachloropropane was 94 % (yield of 94%) and the concentration of Al was 1 ppm.
(2) 1, 1, 1, 2, 3-pentachloropropane was then distilled. Water and a low-boiling point material were removed at a temperature at the bottom of the column of 125°C or lower under a reduced pressure at the top of the column of 10 kPa. After water and the low-boiling point material were removed, distillation was carried out at a maximum temperature at the bottom of the column of 150°C under a reduced pressure at the top of the column of 10 kPa to obtain 2,700 g of 1,1,1,2,3-pentachloropropane having a purity of not less than 99.5 % as an effluent from the top of the column. The decomposition rate of 1,1,1,2,3-pentachloropropane was not more than 0.1%. The bottom of the column after distillation was clean without a deposit.

To simulatively create the state of the bottom of the column when distillation was carried out continuously for a long time, the following experiment was conducted for evaluation. 50 g of the residual solution remaining at the bottom of the column after the above distillation was taken out and put into a 100-ml flask together with an SUS plate having a surface area of about 16 cm². After 24 hours of agitation at a temperature of 150°C and about 15 kPa, the SUS plate was taken out and vacuum dried. As a result, it was confirmed that the weight of the SUS plate increased by 0.9 mg.

### Example 2

(1) After a dehydrochlorination reaction and a fluorination reaction were carried out in the same manner as in Example 1 and an inflow of chlorine was stopped, the concentration of hydrogen chloride contained in the reaction solution was measured. The concentration was 4,200ppmw based on the solution after the reactions. 200 g of ethylene glycol was added in place of water under agitation and an ethylene glycol layer (light layer) was separated to obtain 2,855 g of a reaction solution (heavy layer) after extraction. The ethylene glycol layer contained no solid. When the reaction solution was analyzed by GC and ICP, the conversion rate of 1,1,1,3-tetrachloropropane was 100 %, the selectivity of 1,1,1,2,3-pentachloropropane was 94 % (yield of 94 %) and the concentration of Al was 1 ppm.
(2) 1,1,1,2,3-pentachloropropane was then distilled. A low-boiling point material was removed at a temperature at the bottom of the column of 125°C or lower under a reduced pressure at the top of the column of 10 kPa. After the low-boiling point material was removed, distillation was carried out at a maximum temperature at the bottom of the column of 150°C under a reduced pressure at the top of the column of 10 kPa to obtain 2,690 g of 1,1,1,2,3-pentachloropropane having a purity of not less than 99 % as an effluent from the top of the column. The decomposition rate of 1,1,1,2,3-pentachloropropane was not more than 0.1%. The bottom of the column after distillation was clean without a deposit.

To simulatively create the state of the bottom of the column when distillation was carried out continuously for a long time, the following experiment was conducted for evaluation. 50 g of the residual solution remaining at the bottom of the column after the above distillation was taken out and put into a 100-ml flask together with an SUS plate having a surface area of about 16 cm². After 24 hours of agitation at a temperature of 150°C and about 15 kPa, the SUS plate was taken out and vacuum dried. As a result, it was confirmed that the weight of the SUS plate increased by 1 mg.

### Example 3

(1) After a dehydrochlorination reaction and a fluorination reaction were carried out in the same manner as in Example 1 and an inflow of chlorine was stopped, the concentration of hydrogen chloride contained in the reaction solution was measured. The concentration was 4,100ppmw based on the solution after the reactions. Thereafter, 200g of propylene glycol was added in place of water under agitation and a propylene glycol layer (light layer) was separated to obtain 2, 860 g of a reaction solution (heavy layer) after extraction. When the reaction solution was analyzed by GC and ICP, the conversion rate of 1,1,1,3-tetrachloropropane was 100 %, the selectivity of 1,1,1,2,3-pentachloropropane was 94 % (yield of 94 %) and the concentration of Al was 2 ppm.
(2) 1,1,1,2,3-pentachloropropane was then distilled. A low-boiling point material was removed at a temperature at the bottom of the column of 125°C or lower under a reduced pressure at the top of the column of 10 kPa. After the low-boiling point material was removed, distillation was carried out at a maximum temperature at the bottom of the column of 150°C under a reduced pressure at the top of the column of 10 kPa to obtain 2,700 g of 1,1,1,2,3-pentachloropropane having a purity of not less than 99% as an effluent from the top of the column. The decomposition rate of 1,1,1,2,3-pentachloropropane was not more than 0.1%. The bottom of the column after distillation was clean without a deposit.

### Example 4

1, 820 g of purified 1,1,1,3-tetrachloropropane having a purity of 99.5% was fed to a 2, 000-ml four-necked eggplant flask. Then, 1.0 g of anhydrous iron chloride was further fed to the flask and stirred for 1 hour by setting the liquid temperature to 20°C. After it was confirmed that the solution turned yellow and iron chloride dissolved, the liquid temperature was set to 65°C and chlorine was caused to flow into the flask at 800 ml/min. After 6 hours, an inflow of chlorine was stopped, and nitrogen was let pass through the flask to remove chlorine. Thereafter, 300 g of water was put into the flask to be mixed and a water layer (light layer) was separated by stopping agitation to obtain 2,200 g of a reaction solution (heavy layer). No solid was seen in the water layer. When the reaction solution was analyzed by ICP and GC, the conversion rate of 1,1,1,3-tetrachloropropane was 100 %, the selectivity of 1,1,1,2,3-pentachloropropane was 65 % (yield of 65 %) and the concentration of Fe was not more than 1 ppm.

1,1,1,2,3-pentachloropropane was then distilled. Water and a low-boiling point material were removed at a temperature at the bottom of the column of 125°C or lower under a reduced pressure at the top of the column of 10 kPa. After water and the low-boiling point material were removed, distillation was carried out at a maximum temperature at the bottom of the column of 150°C under a reduced pressure at the top of the column of 10 kPa to obtain 1,410 g of 1,1,1,2,3-pentachloropropane as an effluent from the top of the column. The bottom of the column after distillation was clean without a deposit.

To simulatively create the state of the bottom of the column when distillation was carried out continuously for a long time, the following experiment was conducted for evaluation. 50 g of the residual solution remaining at the bottom of the column after the above distillation was taken out and put into a 100-ml flask together with an SUS plate having a surface area of about 16 cm². After 24 hours of agitation at a temperature of 150°C and about 15 kPa, the SUS plate was taken out and vacuum dried. As a result, it was confirmed that the weight of the SUS plate increased by 0.9 mg.

### Comparative Example 1

(1) 2,387 g of purified 1,1,1,3-tetrachloropropane having a purity of 99.5 wt% was fed to a 2,000-ml eggplant flask as in Example 1. Then, 0. 62 g of anhydrous aluminum chloride was fed to the flask and stirred by setting the liquid temperature to 30°C. After it was confirmed that the solution turned blue and aluminum chloride dissolved, chlorine was caused to flow into the flask at a rate of 2,000 ml/min while the liquid temperature was kept at 25 to 30°C. After 1.5 hours, chlorine was caused to flow at a rate of 1,000 ml/min. After 45 minutes, the flow rate of chlorine was further changed to 500 ml/min. An inflow of chlorine was stopped after 30 minutes, and 1 ml of eugenol was added. Nitrogen was supplied to set the chlorine and hydrogen chloride concentrations to not more than 100 ppm. When the reaction solution was analyzed by GC and ICP, the conversion rate of 1,1,1,3-tetrachloropropane was 100 %, the selectivity of 1,1,1,2,3-pentachloropropane was 94 % (yield of 94 %) and the concentration of Al was 50 ppm.
(2) 1,1,1,2,3-pentachloropropane was then distilled. A low-boiling point material was removed at a temperature at the bottom of the column of 125°C or lower under a reduced pressure at the top of the column of 10 kPa and then distillation was carried out at a maximum temperature at the bottom of the column of 140°C under a reduced pressure at the top of the column of 10 kPa to obtain 2,745 g of 1,1,1,2,3-pentachloropropane having a purity of not less than 99.5 % as an effluent from the top of the column. The decomposition rate of 1,1,1,2,3-pentachloropropane was not more than 0.1 %. A small amount of a deposit was seen at the bottom of the column after distillation.

To simulatively create the state of the bottom of the column when distillation was carried out continuously for a long time, the following experiment was conducted for evaluation. 50 g of the residual solution remaining at the bottom of the column after the above distillation was taken out and put into a 100-ml flask together with an SUS plate having a surface area of about 16 cm². After 24 hours of agitation at a temperature of 150°C and about 15 kPa, the SUS plate was taken out and vacuum dried. As a result, it was confirmed that the weight of the SUS plate increased by 24 mg.

It was understood from the above simulative experiment that, according to the present invention, when distillation is carried out continuously at 150°C, the amount of the deposit can be reduced to not more than 1/24 of the above amount.

### Comparative Example 2

Reactions and distillation were carried out in the same manner as in Comparative Example 1. To simulatively create the state of the bottom of the column when distillation was carried out continuously for a long time, the following experiment was conducted for evaluation. 50 g of the residual material remaining at the bottom of the column after the above distillation was taken out and put into a 100-ml flask together with an SUS plate having a surface area of about 16 cm². After 24 hours of agitation at a temperature of 150°C and about 15 kPa, the SUS plate was taken out and vacuum dried. As a result, it was confirmed that the weight of the SUS plate increased by 4 mg.

It is understood from the above simulative experiment that, according to the present invention, even when distillation is carried out continuously at a high temperature of 150°C as in Example 1, the amount of a deposit can be reduced to about 1/4 of the amount of a deposit when distillation is carried out continuously at 130°C.

### Effect of the Invention

According to the present invention, when the reaction solution obtained by a chlorination reaction is purified by distillation, the yield of the high-order chloroalkane of interest can be greatly improved and distillation operation can be carried out continuously and stably for a long time. Further, according to the present invention, since a side reaction can be prevented even when a high distillation temperature and a high distillation pressure are set, the production method of the present invention is efficient and industrially beneficial from the viewpoints of distillation equipment cost, operation cost and time savings.

## Claims

1. A high-order chloroalkane production method comprising the steps of:
carrying out the dehydrochlorination reaction of a chloroalkane represented by the following formula (1) in the presence of a Lewis acid catalyst of a metal chloride and chlorinating the formed chloroalkene to produce a high-order chloroalkane represented by the following formula (2);
removing a metal component derived from the catalyst from the obtained reaction solution; and
distilling the reaction solution to isolate the high-order chloroalkane.
Cₐ₊₁H_{b}Cl_{c+4} (1)
wherein "a" is an integer of 2 to 4, "b" is an integer of 1 to 2a, and "c" is an integer of 0 to (2a-1), with the proviso that (b+c) is 2a.
Cₐ₊₁H_{b-1}Cl_{c+5} (2)
wherein "a", "b" and "c" are as defined in the above formula (1).

2. The high-order chloroalkane production method according to claim 1, wherein chlorine is supplied into the reaction solution in which the dehydrochlorination reaction of the chloroalkane represented by the formula (1) is proceeding to carry out the chlorination reaction of the chloroalkene produced in the reaction solution at the same time so as to produce the high-order chloroalkane represented by the formula (2).

3. The high-order chloroalkane production method according to claim 1 or 2, wherein the removal of the metal component derived from the catalyst is carried out by solvent extraction.

4. The high-order chloroalkane production method according to claim 3, wherein the concentration of hydrogen chloride contained in the reaction solution to be subjected to solvent extraction is 1.0 x 10⁻⁵ to 5.0 x 10⁻² part by mass based on 1 part by mass of the high-order chloroalkane represented by the formula (2).

5. The high-order chloroalkane production method according to claim 3 or 4, wherein an extraction solvent for solvent extraction is water or a polyhydric alcohol.

6. The high-order chloroalkane production method according to claim 5, wherein the polyhydric alcohol is ethylene glycol, propylene glycol or glycerin.

7. The high-order chloroalkane production method according to any one of claims 1 to 6, wherein the metal chloride is anhydrous aluminum chloride.

8. The high-order chloroalkane production method according to any one of claims 1 to 7, wherein the distillation of the reaction solution from which the metal component derived from the catalyst has been removed is carried out at a temperature at the bottom of a distillation column of 100 to 300°C.

9. The high-order chloroalkane production method according to any one of claims 1 to 8, wherein the concentration of the metal component derived from the catalyst remaining in the reaction solution to be distilled is not more than 5.0 x 10⁻⁶ part by mass based on 1 part by mass of the high-order chloroalkane represented by the formula (2).

10. The high-order chloroalkane production method according to any one of claims 1 to 9, wherein the chloroalkane represented by the formula (1) is 1,1,1,3-tetrachloropropane and the high-order chloroalkane represented by the formula (2) is 1,1,1,2,3-pentachloropropane.
